(19) Europäisches Patentamt
European Patent Office
Office européen des brevets

(11) Numéro de publication: 0 349 443
A1

(12) # DEMANDE DE BREVET EUROPEEN

(21) Numéro de dépôt: 89402058.5

(22) Date de dépôt: 28.06.89

(51) Int. Cl.5: G 06 K 11/06
B 43 K 29/08, A 61 B 17/32,
G 01 B 7/16, G 01 L 1/22,
G 06 K 9/22

(30) Priorité: 30.06.88 FR 8808879

(43) Date de publication de la demande:
03.01.90 Bulletin 90/01

(84) Etats contractants désignés: ES GR

(71) Demandeur: Fischer, Guy
6, Rue Henri Dunant
F-95400 Arnouville les Gonesse (FR)

(72) Inventeur: Fischer, Guy
6, Rue Henri Dunant
F-95400 Arnouville les Gonesse (FR)

(54) **Outil à main muni de moyens de détection de la flexion de l'élément d'extrémité.**

(57) L'invention concerne un instrument à main du type comprenant un manche (2) et un organe (4) propre à venir en coopération quasi-ponctuelle avec une surface.

Selon une définition générale de l'invention, l'une des extrémités de l'organe (6) est encastrée dans le manche (2) tandis que l'autre extrémité (14), libre, comprend des moyens capteurs (16) propres à capter la flexion de l'organe, en tant que grandeur représentative de la pression exercée par l'organe sur ladite surface.

16
10
26
20
14
4
26
12
8
6
FIG.2

EP 0 349 443 A1

## Description

### Outil à main sensible à sa coopération quasi-ponctuelle avec une surface

L'invention concerne un outil ou instrument à main sensible à sa coopération quasi-ponctuelle avec une surface. Elle trouve une application dans le traitement des informations produites par ladite sensibilité de l'instrument à sa coopération quasi-ponctuelle avec une surface. Par exemple, elle s'applique à l'authentification de signature, la reconnaissance de l'écriture manuscrite, l'aide à l'écriture, et l'aide à l'incision dans une chair, etc.

On connaît déjà des stylos à bille sensibles à la coopération quasi-ponctuelle de la bille avec une feuille de papier en vue de reconnaître l'écriture manuscrite ou de vérifier une signature.

Généralement, ce genre de stylo comprend des moyens transducteurs piezo-électriques capacitifs ou inductifs couplés à la bille et propres à déterminer la force d'application de la bille sur la feuille de papier ainsi que son changement de direction.

De tels stylos ne sont pas satisfaisants car les moyens transducteurs sont de mise en oeuvre difficile, d'une part, et ces stylos s'appliquent uniquement à la vérification de signature ou la reconnaissance d'écriture manuscrite, d'autre part.

L'invention vise à apporter une solution à ce problème.

L'invention porte sur un instrument à main de type comprenant un manche et un organe propre à venir en coopération quasi-ponctuelle avec une surface.

Selon une définition générale de l'invention, l'une des extrémités de l'organe est encastrée dans le manche tandis que l'autre extrémité, libre, comprend des moyens capteurs propres à capter la flexion de l'organe, en tant que grandeur représentative de la pression exerçée par l'organe sur la surface.

Selon un aspect de l'invention, les moyens capteurs comprennent au moins une jauge de contrainte disposée sur l'une des faces de l'organe, la jauge de contrainte délivrant un signal électrique en réponse à la flexion de l'organe et l'outil est branché à des moyens de traitement propres à traiter le signal ainsi délivré par la jauge de contrainte, en vue de calculer les paramètres relatifs à la flexion de l'organe et d'obtenir des images représentatives de la coopération quasi-ponctuelle de l'organe avec la surface.

Selon un mode de réalisation préféré de l'outil selon l'invention, les moyens capteurs comprennent deux paires de jauges de contrainte disposées chacune sur chaque face de l'organe, lesdites paires de jauges de contrainte étant agencées selon un montage électrique du type pont de Wheatstone, de manière à obtenir un signal électrique représentatif de la flexion de l'organe.

Avantageusement, les jauges de contrainte sont collées sur les faces de l'organe.

Selon un autre mode de réalisation préféré de l'invention, les jauges de contrainte sont implantées sur l'organe par diffusion directe des jauges sur ledit organe.

Selon une autre caractéristique de l'invention, les moyens de traitement comprennent :
- un organe convertisseur analogique-numérique propre à rendre numérique le signal électrique délivré par les moyens capteurs, ledit organe convertisseur possédant une entrée reliée aux moyens capteurs et une sortie ;
- des moyens de mémoire propres à stocker les signaux ainsi numérisés, lesdits moyens de mémoire possédant une entrée reliée à la sortie de l'organe convertisseur analogique-numérique et au moins une sortie ;
- des moyens de traitement de l'information numérique propre à traiter les signaux numériques ainsi mémorisés en vue de calculer les paramètres relatifs à la flexion de l'organe et d'obtenir des images représentatives de la coopération de l'organe avec la surface, lesdits moyens de traitement d'informations numériques possédant une entrée reliée à la sortie des moyens de mémoire et au moins une sortie.

Selon un mode de réalisation préféré de l'invention selon lequel l'organe est propre à venir en coopération du genre écriture avec une surface, le manche est le corps d'un stylo tandis que l'organe est la plume dudit stylo.

Selon un autre mode de réalisation préféré de l'invention selon lequel l'organe est propre à venir en coopération du genre incision avec une chair, le manche est le corps d'un bistouri tandis que l'organe est la lame dudit bistouri.

D'autres avantages et caractéristiques de l'invention apparaîtront à l'examen de la description détaillée ci-après et des dessins annexés dans lesquels :
- la figure 1 représente schématiquement une vue de dessus de l'instrument selon l'invention ;
- la figure 2 représente schématiquement une vue en coupe longitudinale de l'instrument selon l'invention ;
- la figure 3 représente une vue partielle de l'instrument au repos selon l'invention ;
- la figure 4 représente une vue partielle de l'instrument en coopération quasi-ponctuelle avec une surface ;
- la figure 5 représente schématiquement le montage électrique des deux paires de jauges de contrainte disposées chacune sur chaque face de l'organe ;
- la figure 6 représente le schéma-bloc des éléments essentiels constitutifs de l'instrument à main selon l'invention ; et
- la figure 7 représente schématiquement un exemple d'application de l'instrument à main sous la forme d'un stylo.

Les dessins annexés comportent de nombreux éléments de caractère certain qu'ils peuvent seuls apporter. Ils font donc partie intégrante de la description, pour servir à faire comprendre l'invention, aussi bien qu'à la définir.

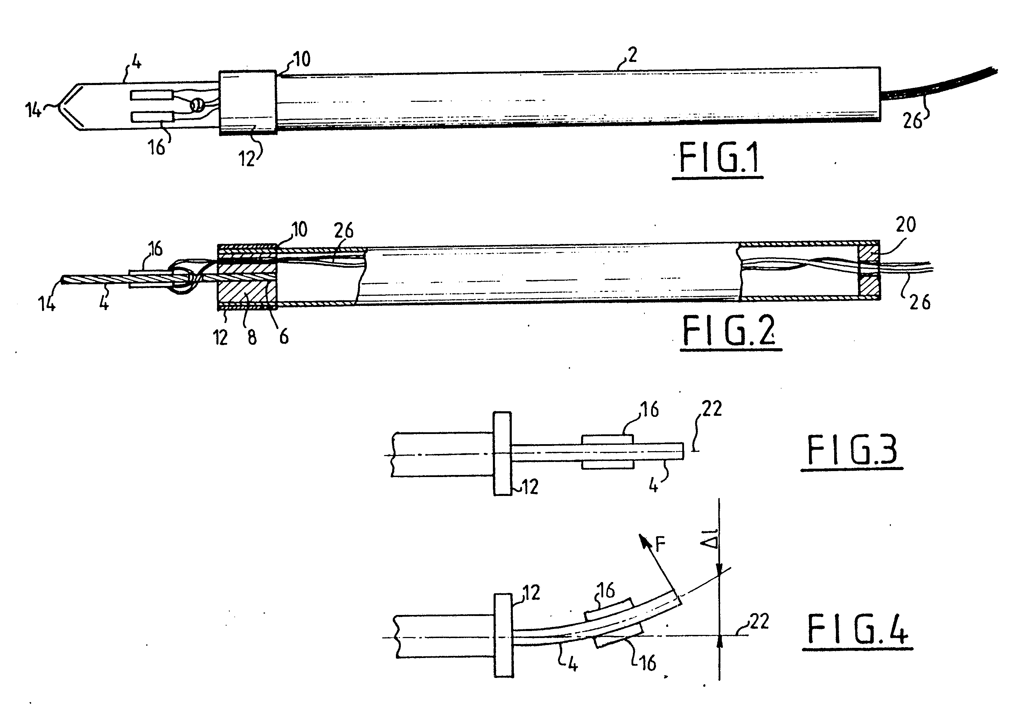

L'instrument à main selon l'invention (figures 1 et 2) est du genre comprenant un manche allongé cylindrique 2 et un organe telle qu'une lame rectangulaire plate 4 propre à venir en coopération quasi-ponctuelle avec une surface.

Le manche 2 est creux de manière à permettre le passage de liaisons électriques que l'on décrira ci-après.

L'une des extrémités 6 de la lame 4 est encastrée dans une pièce cylindrique 8 bouchant l'une des extrémités 10 du manche 2. Une bague 12 maintient serrée l'extrémité de la lame 6 dans la pièce 8.

L'autre extrémité 14 de la lame 2, qui vient en coopération quasi-ponctuelle avec une surface, est effilée en forme de pointe.

Sur chaque face de la partie libre de la lame 4, sont disposées deux paires de jauges de contrainte rectangulaires plates 16.

Chaque paire de jauges de contrainte 16 est collée sur la face respective de la lame par l'intermédiaire d'une colle de type acrylique. La lame 4 est, par exemple, une plaque rectangulaire métallique, de l'aluminium, par exemple, poli par un moulage fin, puis traité par un bain à trois acides, et enfin dégraissé.

La plaque métallique 4 peut être remplacée par un tube cylindrique métallique.

Les jauges de contrainte 16 sont reliées électriquement par des liaisons électriques 26 qui traversent longitudinalement le manche et qui sortent à l'autre extrémité du manche 20.

Les jauges 16 sont à fil résistif ou à trames pelliculaires.

Sur la figure 3, on a représenté une vue partielle de l'instrument à main au repos. On y retrouve la lame 4, munie de ses jauges de contrainte 16. L'axe 22 de la lame 4, encastrée dans le manche 2, est parallèle à celui du manche 2.

Sur la figure 4, on a représenté une vue partielle de l'instrument à main dont la lame 4 est en coopération quasi-ponctuelle avec une surface (non représenté).

La pression F exerçée par la lame 4 sur la surface fait fléchir la lame d'un angle $\Delta$ L par rapport à l'axe 22 de la lame au repos. Les deux paires de jauges disposées sur chaque face de la lame captent la flexion de la lame qui provoque une variation de la résistance des jauges. En réponse à ladite flexion les jauges délivrent un signal électrique proportionnel à l'angle $\Delta$ L de la flexion de la lame.

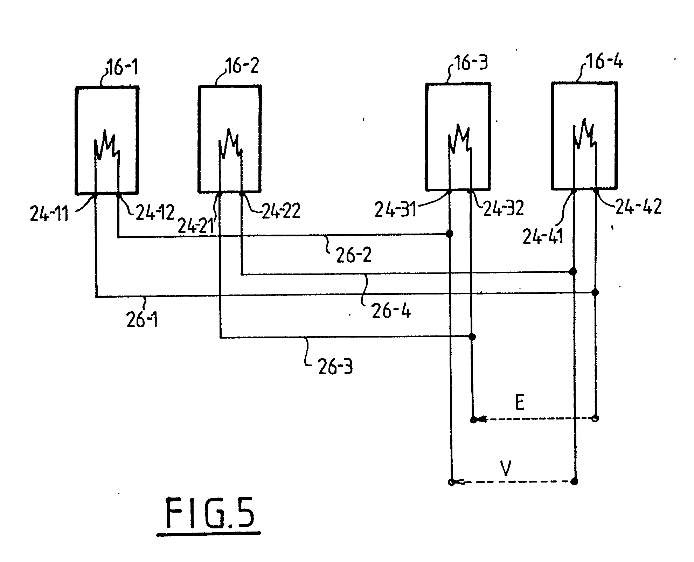

Sur la figure 5, on a représenté un exemple de montage électrique de la paire de jauges 16-1,16-2 disposées sur l'une des faces de la lame et de la paire de jauges 16-3,16-4 disposées sur l'autre des faces de la lame.

Chaque jauge 16 possède deux bornes électriques 24, par exemple, la jauge 16-1 possède une borne 24-11 et une borne 24-12, etc.

Le montage électrique des quatre jauges est du type pont de Wheatstone. La borne 24-11 est reliée par une liaison électrique 26-1 à la borne 24-42. La borne 24-12 est reliée par une liaison 26-2 à la borne 24-31. La borne 24-21 est reliée par l'intermédiaire d'une liaison 26-3 à la borne 24-32. La borne 24-22 est reliée par l'intermédiaire d'une liaison électrique 26-4 à la borne 24-41.

On établit une différence de potentiel nulle entre la borne 24-42 et la borne 24-32, tandis que la différence de potentiel représentative de la flexion de la lame est obtenue entre les bornes 24-41 et 24-31.

Des résultats ont été obtenus dans les conditions expérimentales suivantes.

Les jauges étaient alimentées par une alimentation alternative délivrant une tension alternative de 3,5 volts efficaces.

Pour une flexion $\Delta$ L de l'ordre de 0,5 mm, un signal électrique de l'ordre de 1 volt était obtenu après conditionnement, démodulation synchrone et gain convenablement choisi.

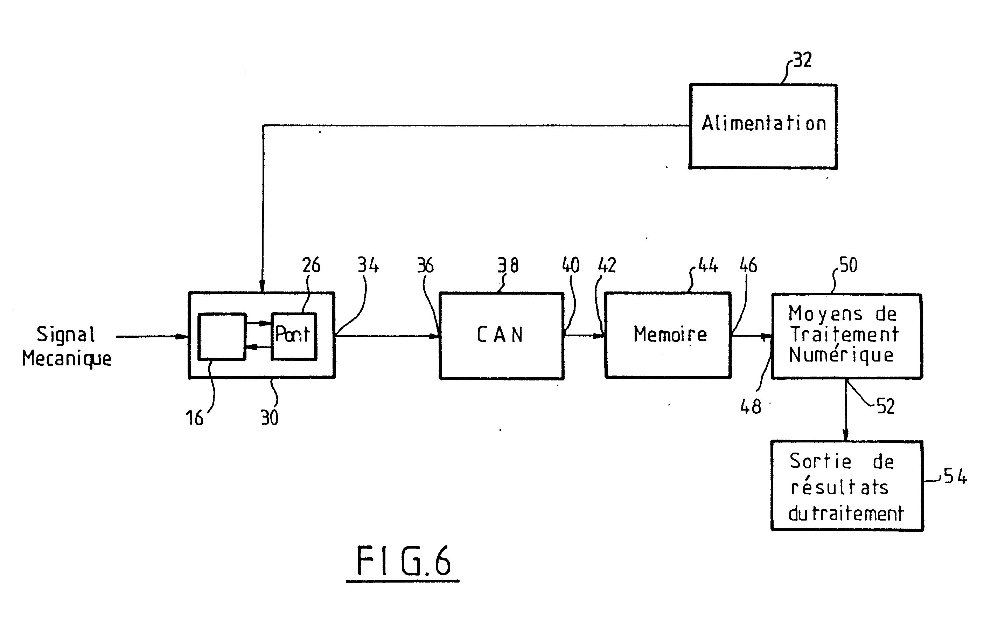

Sur la figure 6, on a représenté le schéma-bloc de l'instrument à main relié à des moyens de traitement par l'intermédiaire des liaisons électriques 26 reliant les bornes des jauges et traversant le manche de l'instrument.

Le capteur 30, constitué des jauges 16, équilibré selon un montage électrique de type pont de Wheatstone, est alimenté par des moyens d'alimentation 32 délivrant une tension qui constitue la source d'activation du pont.

La source d'activation du pont de Wheatstone peut être alternative comme dans le cas du prototype décrit ci-avant mais peut également être continue, voire en impulsions, les conditionneurs étant, bien entendu, légèrement différents.

Le signal mécanique, produit par la coopération de la lame 4 avec la surface, sollicite les jauges 16 qui délivrent un signal électrique en réponse au signal mécanique.

Le capteur 30 possède une sortie 34 reliée à l'entrée 36 d'un circuit convertisseur analogique-numérique 38.

Par exemple, le circuit convertisseur analogique-numérique 38 échantillonne le signal électrique délivré par le capteur 34 a une fréquence d'échantillonnage de 100 hertz.

Le convertisseur analogique-numérique 38 possède une sortie 40 reliée à l'entrée 42 d'une mémoire 44 propre à stocker le signal délivré par les jauges 16 ainsi numérisé par le circuit convertisseur analogique-numérique 38.

La mémoire 44 possède une sortie 46 reliée à l'entrée 48 de moyens de traitement numérique 50 propres à calculer les paramètres relatifs à la flexion de la lame et propres à obtenir des images représentatives de la coopération de la lame avec la surface.

Les moyens de traitement numérique 50 possèdent une sortie 52 délivrant des résultats de traitement numérique 54. Les moyens de traitement numérique peuvent piloter des périphériques telles qu'une imprimante, un écran, etc.

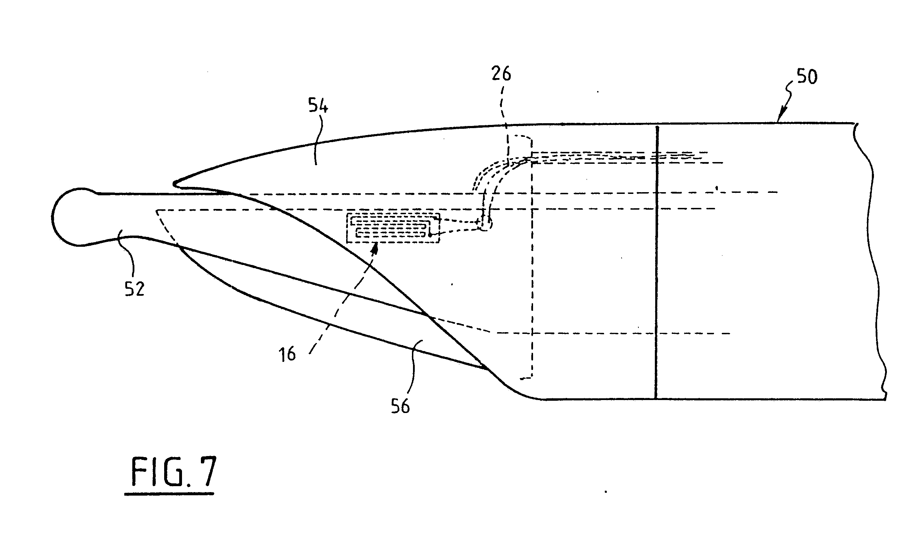

Sur la figure 7, on a représenté une utilisation de l'instrument à main sous la forme d'un stylo à plume. Le stylo à plume comprend un corps 50 et une plume 52 insérée entre le bec supérieur 54 et le bec inférieur 56 dudit stylo.

Une jauge de contrainte 16 est disposée sur chaque face interne du bec supérieur 54. Des liaisons électriques 26 relient les jauges de contrainte 16 aux moyens de traitement numérique décrits ci-avant.

Un tel stylo à plume peut être utilisé comme authentifi cateur de signatures.

Dans cette utilisation, le stylo à plume capte l'évolution de pression de la plume au cours de la signature. Les moyens de traitement numérique comparent le signal numérique obtenu à la signature avec un signal numérique de référence pré-enregistré dans la mémoire.

Par exemple, l'information brute recueillie lors d'un tracé manuscrit est constituée d'une série de 300 à 500 valeurs codées, chacune sur un ou plusieurs octets. Les moyens de traitement font subir à ces valeurs alors un traitement mathématique destiné à normaliser l'échantillon recueilli et à compresser l'information pour qu'elle n'occupe plus qu'un espace mémoire réduit (50 à 150 octets). Le résultat ainsi obtenu subit un traitement informatique (du type de ceux utilisés dans le traitement de la parole) puis est comparé à un modèle mémorisé, figurant par exemple dans une carte à puce.

On obtient alors une identification fiable du porteur attitré d'une carte à puce, par exemple, carte de crédit.

L'instrument à main peut être utilisé pour reconnaître en temps réel l'écriture manuscrite. Cette utilisation, qui est proche de la précédente, consiste à un traitement informatisé comparant chaque caractère tracé, associé à un signal mécanique proportionnel au temps, avec un alphabet mémorisé. Ce traitement informatique permet d'identifier le caractère tracé ainsi comparé avec un alphabet mémorisé de référence.

L'instrument à main peut être également utilisé avec une table à digitaliser en vue de moduler la largeur de traits en fonction de la pression exercée.

Dans cette utilisation, le capteur est utilisé couplé avec une table à digitaliser à contact mécanique. Le manipulateur trace des traits à l'aide d'un outil à main comprenant une lame sensible à sa coopération avec une table à digitaliser. Les signaux délivrés par l'outil à main sont traités par des moyens de traitement numérique couplés à un écran en vue de représenter à l'écran des images représentatives de ladite coopération de la lame avec la table à digitaliser. Ainsi, on obtient à l'écran un tracé dont l'épaisseur est proportionnelle à la force exerçée par la lame sur la table à digitaliser. Un tel instrument à main permet ainsi d'obtenir à l'écran un trait de largeur variable que l'on peut moduler à volonté ce qui ouvre la porte à des applications diverses, artistiques par exemple.

Enfin, l'instrument à main peut être utilisé dans une application médicale, selon laquelle la lame de l'outil à main vient en coopération du genre incision avec une chair.

Avec un bistouri comprenant une lame dont la partie tranchante forme un angle droit avec la partie équipée de jauges de contrainte, comme décrit ci-avant, le chirurgien est à même de connaître, en permanence, la pression qu'il exerce avec son bitouri sur une chair, ce qui permet également de mieux contrôler la profondeur de l'entaille.

On peut aussi équiper la butée d'un bistouri ophtalmique de jauges de contrainte, comme décrit ci-avant. Ces jauges de contrainte fournissent une indication précise de la profondeur d'entaille qui est fonction de la déformation de la butée.

La butée peut, en même temps, servir de limitateur de pénétration du bistouri en donnant une indication de la pression qu'elle exerce sur une surface à inciser. Cette indication permet d'éviter une déformation de la surface qui laisserait le bistouri atteindre des zones de profondeur indésirée. La lame, sensible à sa coopération avec un tissu, chair, organe, se déformant avec les sollicitations de la butée, doit être adaptée (étalonnée) en sensibilité à la nature du tissu entaillé.

**Revendications**

1. - Instrument à main du type comprenant un manche (2) et un organe (4) propre à venir en coopération quasi-ponctuelle avec une surface, caractérisé en ce que l'une des extrémités de l'organe (6) est encastrée dans le manche (2) tandis que l'autre extrémité (14), libre, comprend des moyens capteurs (30) propres à capter la flexion de l'organe, en tant que grandeur représentative de la pression exerçée par l'organe sur ladite surface.

2. - Instrument à main selon la revendication 1, caractérisé en ce que les moyens capteurs (30) comprennent au moins une jauge de contrainte (16) disposée sur l'une des faces de l'organe, la jauge de contrainte délivrant un signal électrique en réponse à la flexion de l'organe et en ce que l'instrument est branché à des moyens de traitement propres à traiter le signal ainsi délivré par la jauge de contrainte (16) en vue de calculer les paramètres relatifs à la flexion de l'organe et d'obtenir des images représentatives de la coopération dudit organe avec la surface.

3. - Instrument à main selon la revendication 2, caractérisé en ce que les moyens capteurs (30) comprennent deux paires de jauges de contrainte (16) disposées chacune sur chaque face de l'organe (4), lesdites paires de jauges de contrainte étant agencées selon un montage électrique du type pont de Wheatstone, de manière à obtenir un signal électrique représentatif de la flexion de l'organe.

4. - Instrument à main selon l'une des revendications 2 et 3, caractérisé en ce que les jauges de contrainte sont collées sur les faces de l'organe.

5. - Instrument à main selon l'une des revendications 2 et 3, caractérisé en ce que les jauges de contrainte sont implantées sur les faces de l'organe par diffusion directe des jauges sur ledit organe.

6. - Instrument à main selon la revendication 3, caractérisé en ce que les moyens de traitement comprennent :

- un organe convertisseur analogique-numérique (38) propre à numériser le signal délivré par les moyens capteurs (30), ledit organe (38) possédant une entrée (36) reliée aux moyens capteurs (30) et une sortie (40) ;
- des moyens de mémoire (44) propres à stocker les signaux ainsi numérisés, lesdits moyens de mémoire (44) possédant une entrée (42) reliée à la sortie (40) et une sortie (46) ;
- des moyens de traitement numérique (50) propres à traiter les signaux numériques délivrés par l'organe (38) pour calculer les paramètres relatifs à la flexion de l'organe et pour obtenir une image de la coopération de l'organe avec une surface, lesdits moyens de traitement numérique possédant une entrée (48) reliée à la sortie des moyens de mémoire (46) et une sortie (52).

7. - Instrument à main selon lequel l'organe est propre à venir en coopération, du genre écriture, avec une surface, selon l'une quelconque des précédentes revendications, caractérisé en ce que le manche est le corps d'un stylo, tandis que l'organe est la plume dudit stylo.

8. - Instrument à main selon lequel l'organe est propre à venir en coopération, du genre incision dans une chair, selon l'une des revendications 1 à 6, caractérisé en ce que le manche est le corps d'un bistouri, tandis que l'organe est la lame dudit bistouri.

FIG.1
FIG.2
FIG.3
FIG.4
2
4
6
8
10
12
14
16
20
22
26
F

16-1
16-2
16-3
16-4
24-11
24-12
24-21
24-22
24-31
24-32
24-41
24-42
26-2
26-4
26-1
26-3
E
V

FIG.5

Alimentation
32
Moyens de Traitement Numérique
50
52
48
Sortie de résultats du traitement
54
Memoire
44
46
42
40
CAN
38
36
34
26
Pont
30
16
Signal Mecanique
FIG.6

50
26
54
56
16
52

FIG. 7

Office européen des brevets

# RAPPORT DE RECHERCHE EUROPEENNE

Numero de la demande

EP 89 40 2058

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A- 411 052 (A. SNIDERMAN et al.) * Colonne 1, ligne 60 - colonne 2, ligne 16; figures 1,6 * --- | 1-3,7 | G 06 K 11/06<br>B 43 K 29/08<br>A 61 B 17/32<br>G 01 B 7/16<br>G 01 L 1/22<br>G 06 K 9/22 |
| A | US-A-4 078 226 (E.P. EERNISSE et al.) * Colonne 2, ligne 54 - colonne 3, ligne 20; figures 1,3 * --- | 1,2,6,7 | |
| A | US-A-2 761 216 (F. GOLLUB) * En entier * ----- | 1-4,6 | |

DOMAINES TECHNIQUES RECHERCHES (Int. Cl.5)

G 06 K 11
A 61 B 17
G 01 B 7
G 06 K 9
G 01 L 1
G 01 L 5
A 61 B 17
B 43 K
A 61 B 5

Le présent rapport a été établi pour toutes les revendications

| Lieu de la recherche | Date d'achèvement de la recherche | Examinateur |
|---|---|---|
| LA HAYE | 20-09-1989 | CIARELLI N. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)